# DEMANDE DE BREVET EUROPEEN

(11) **EP 1 454 913 A1**
(43) Date de publication de la demande: **08.09.2004**
(21) Numéro de dépôt: 04290374.0
(22) Date de dépôt: 12.02.2004
(51) Int. Cl.: C07H 13/06

(54) **Procédé de préparation de dérivés O-acylés du glucose**

(30) Priorité: 04.03.2003 FR 0302635
(71) Demandeur: L'OREAL, 75008 Paris (FR)
(72) Inventeur: Henrion, Jean-Christophe, 93500 Pantin (FR); Philippe, Michel, 91320 Wissous (FR)
(74) Mandataire: Dodin, Catherine

(57) **Abrégé**

La présente demande concerne un procédé de préparation de dérivés O-acylés du glucose, notamment majoritairement en position 6, par réaction d'un acide carboxylique avec un halogénoformiate d'alkyle de manière à former un anhydride mixte, puis réaction dudit anhydride mixte avec le glucose.

## Description

La présente invention a trait à un nouveau procédé de préparation de dérivés O-acylés, notamment majoritairement en position 6, du glucose.

Les dérivés O-acylés du glucose sont, d'une manière générale, déjà connus dans l'art antérieur, qui en décrit plusieurs synthèses.
Plusieurs méthodes d'estérification du D-glucose avec l'acide laurique ont été décrites et comparées dans la revue "Die Stärke", n°6, p.181-189 en 1968 par Reinefeld et al. Parmi les agents d'acylation du glucose, il a ainsi été proposé d'employer des chlorures d'acides tels que le chlorure de lauroyle, des imidazolides d'acides, notamment d'acide laurique ou encore des anhydrides mixtes carboxyliques-carboniques.
Il ressort de cette publication que la méthode permettant l'obtention du rendement le plus élevé est l'acylation à l'aide du chlorure d'acide. Avec le chlorure de lauroyle, on obtient par exemple un mélange de monoester et de diesters, avec un rendement de 49% dont 36% pour le monoester. Toutefois, il n'est pas toujours aisé de disposer du chlorure d'acide adéquat. En l'absence de chlorure d'acide industriel, il est alors nécessaire d'utiliser une autre méthode.
L'acylation employant des imidazolides d'acide conduit à un mélange de monoester et de diesters, avec un rendement total de 22% et un rendement de 9% pour le monoester seul, lorsque l'on utilise l'imidazolide d'acide laurique.
Ce document décrit également un procédé de préparation de D-glucopyranose-6-laurinate, dans lequel, dans une première étape, on fait réagir de l'acide laurique avec un chloroformiate d'éthyle, en présence de triéthanolamine, dans le benzène, de manière à former un anhydride mixte, puis, dans une seconde étape, à faire réagir ledit anhydride mixte avec le D-glucose, dans la pyridine, à 80°C. On obtient ainsi le composé recherché avec un rendement pouvant aller de 21 à 28%, selon les quantités relatives des réactifs de départ. Toutefois, la réaction est effectuée dans le benzène, solvant difficilement utilisable au niveau industriel puisque interdit dans certains pays, ou dans la pyridine à chaud. Les inconvénients autres liés à ce procédé sont d'une part le faible rendement et d'autre part les problèmes de purification liés à l'élimination de l'imidazole.
Par ailleurs, l'acylation du glucose par le biais de la formation d'anhydride vrai conduit aux composés recherchés avec un rendement total de 46% pour le mélange monoester et diesters, et de 28% pour l'obtention du monoester. Ce procédé génère la formation d'acides gras libres qu'il est nécessaire d'éliminer pour conduire à des produits terminaux relativement purs. Or, cette élimination peut s'avérer parfois difficile, au vu de la nature des impuretés; de plus, on cherche généralement à éviter les étapes intermédiaires de purification, qui rallongent inutilement le procédé et qui engendrent des coûts supplémentaires, ceci étant incompatible avec un procédé au niveau industriel.

On a constaté que, quelle que soit la méthode envisagée, l'agent d'acylation choisi et/ou la proportion de chacun des réactifs, l'acylation du glucose conduisait toujours à l'obtention d'un mélange dans lequel on a pu identifier le D-glucopyranose-6-ester, mais également le D-glucopyranose-1,6-diester et le D-glucopyranose-2,6-diester comme produits de réaction coexistants.

On connaît également, par le brevet US5498708, un procédé de préparation d'esters de polyol consistant à faire réagir un acide, par exemple un acide gras, avec un chloroformiate d'alkyle en C1-10 ou d'aryle, dans un milieux 100% aqueux, de manière à former un anhydride, puis à faire réagir ledit anhydride avec un polyol, de manière à former le composé recherché. La réaction est effectuée dans la glace pillée, donc à une température proche de 0°C. Il est à noter que le seul chloroformiate exemplifié est le chloroformiate d'éthyle, présenté dans la description comme réactif plus particulièrement préféré. Ici encore, les conditions opératoires, notamment de manipulation dans la glace pillée, peuvent se révéler difficilement industrialisables.

On connaît encore, par le brevet EP566438, un procédé de préparation de monoesters du D-maltose, consistant à préparer dans un premier temps, un anhydride mixte, par réaction en présence d'une base, d'un acide carboxylique et d'un chloroformiate d'alkyle, puis dans un deuxième temps, à faire réagir ledit anhydride mixte avec le D-maltose.
Grâce à ce procédé, il est possible de manière surprenante d'acyler sélectivement la position 6' du D-maltose, et non la position 6 portée par une unité glucose dont la fonction anomérique est pourtant libre.

Il subsiste donc le besoin de disposer d'une nouvelle voie de synthèse des dérivés O-acylés du glucose, permettant l'obtention de ces composés d'une manière rapide et aisée, au niveau industriel, avec un rendement en produits recherchés important. Or, on a constaté qu'en utilisant des réactifs bien particuliers, il était possible d'atteindre ce but, en effectuant de plus la réaction à une température proche de la température ambiante, de l'ordre de 20-25°C, ce qui permettait d'éviter en outre une étape de chauffage.

La présente invention a donc pour objet un procédé de préparation de dérivés O-acylés du glucose, notamment majoritairement en position 6, de formule (1) : 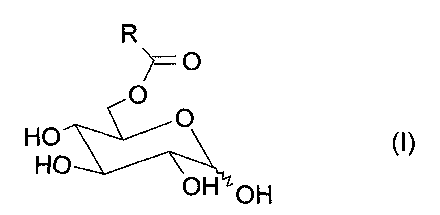 dans laquelle R est une chaîne hydrocarbonée linéaire ou ramifiée, saturée ou insaturée, comprenant 7 à 21 atomes de carbone, consistant:
- dans une première étape, à préparer un anhydride mixte de formule (II) : 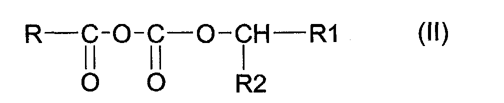 dans laquelle R1 et R2 sont, indépendamment l'un de l'autre, des radicaux hydrocarbonés, linéaires ou ramifiés, saturés ou insaturés, comprenant 1 à 20 atomes de carbone,
   par réaction d'un acide carboxylique de formule R-COOH avec un halogénoformiate d'alkyle de formule X-C(O)-O-CHR1R2, avec X représentant un halogène, de préférence le chlore ou le brome;
- dans une seconde étape, à faire réagir ledit anhydride mixte formé avec le glucose.

Le procédé objet de la présente invention permet de préparer notamment des dérivés O-acylés du glucose majoritairement en position 6, seuls ou en mélange, qui peuvent être représentés par la formule (I).

Ceci est d'autant plus surprenant que, contrairement à ce qui pouvait être attendu par l'homme du métier, à la lecture de EP566438 mentionné plus haut, la réaction s'effectue ici sur la position 6 du glucose, dont la fonction anomérique est libre.

Par ailleurs, cette régiosélectivité en position 6 du glucose peut également paraître inattendue pour l'homme du métier, car on pouvait s'attendre à ce que la fonction OH la plus réactive du glucose soit le OH anomérique hémiacétalique.
De préférence, le radical R est une chaîne hydrocarbonée linéaire ou ramifiée, saturée ou insaturée, comprenant 11 à 17 atomes de carbone.
Le reste acyle -COR peut notamment être un reste octanoyle, décanoyle, dodécanoyle, myristoyle, hexadécanoyle, stéaroyle, palmitoléoyle, oléoyle, linoléoyle ou linolénoyle.
Parmi les acides carboxyliques susceptibles d'être employés pour préparer l'anhydride mixte de formule (II), on peut donc citer les acides octanoïque, décanoïque, dodécanoïque, myristique, hexadécanoïque, stéarique, oléique, linoléique ou linolénique, et leurs mélanges.

Parmi les halogénoformiates d'alkyle de formule X-C(O)-O-CHR1R2, on emploiera plus particulièrement les composés pour lesquels R1 et/ou R2 sont, indépendamment l'un de l'autre, des radicaux hydrocarbonés, linéaires ou ramifiés, saturés ou insaturés, comprenant 1 à 6 atomes de carbone, notamment R1 et/ou R2 sont choisis parmi méthyle ou éthyle, et plus particulièrement les composés X-C(O)-O-CH(CH₃)₂, ou halogénoformiates d'isopropyle, et en particulier le chloroformiate d'isopropyle.

Le schéma réactionnel de la première étape du procédé peut être le suivant :

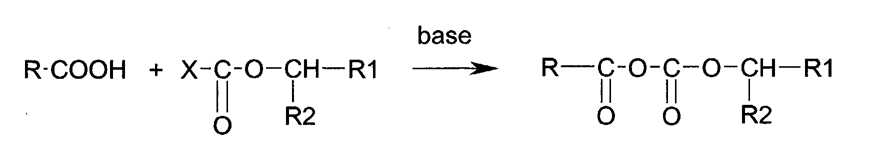

La réaction peut être effectuée dans un solvant organique réactionnel, tel que le tétrahydrofuranne, le N-méthylpyrrolidone, la pyridine, le toluène et leurs mélanges; de préférence dans le toluène.
De préférence, elle peut être effectuée sous atmosphère inerte, azote par exemple.
On peut employer une base pour activer l'acide carboxylique, ou bien utiliser directement le carboxylate correspondant; cette base est, de préférence, une base organique notamment choisie parmi la triéthylamine, la pyridine, la 4-diméthylaminopyridine, la tributylamine, la N-méthylmorpholine et leurs mélanges; de préférence la triéthylamine.
La réaction peut être effectuée à une température de -25°C à +40°C, de préférence -10°C à +10°C, et pendant une durée de 5 minutes à 5 heures, notamment de 30 minutes à 3 heures.
De préférence, on fait réagir 0,3 à 3 équivalents, de préférence 0,5 à 1,5 équivalents, d'acide carboxylique avec 1 équivalent d'halogénure.

Dans la seconde étape du procédé, étape d'estérification, on fait réagir ledit anhydride mixte avec du glucose.
Cette seconde étape peut être éventuellement réalisée après essorage des sels éventuellement formés lors de la première étape.
Cette seconde étape est de préférence effectuée dans un solvant organique, qui peut être le même solvant organique que celui de la première étape. Ce solvant peut donc être le tétrahydrofuranne, le N-méthylpyrrolidone, la pyridine, le toluène et leurs mélanges; de préférence la pyridine.
De préférence, l'anhydride mixte est mis en solution dans ledit solvant organique, avant réaction.
De préférence, le glucose est préalablement mis en solution dans un solvant tel que la pyridine, la N-méthylpyrrolidone et/ou le diméthylacétamide; de préférence la pyridine.
De préférence, on fait réagir 0,5 à 1,5, notamment 0,9 à 1,1, et encore mieux 1, équivalents d'anhydride mixte avec 3 équivalents de glucose.
On utilise de préférence, selon l'invention, un excès de glucose, par exemple au moins 3 équivalents de glucose, par rapport à l'acide ou au mélange d'acides, mis à réagir dans la première étape.
Un avantage de l'invention est que cette deuxième étape du procédé peut être effectuée à une température proche de la température ambiante, par exemple à une température comprise entre 10°C et 40°C, de préférence 15°C à 30°C, et encore mieux 18°C à 25°C.
La réaction peut être conduite pendant une durée de 1 à 15 heures, notamment de 2 à 8 heures.
Après la fin de la réaction, les solvants peuvent être séparés du composé recherché, par exemple par évaporation, centrifugation ou filtration.
Le produit résultant peut être nettoyé par tout moyen connu, tel que distillation, chromatographie sur colonne de gel de silice, précipitation et/ou extraction par exemple par un mélange eau/solvants organiques.

Le procédé selon l'invention permet donc de préparer de manière industriellement réalisable, des dérivés O-acylés du glucose, majoritairement en position 6, seuls ou en mélange. En particulier, on peut préparer selon ce procédé les composés suivants : le 6-O-octadeca-9,12-dienoyl-D-glucopyranose; le 6-O-octadeca-9-enoyl-D-glucopyranose; le 6-O-octadecanoyl-D-glucopyranose; le 6-0-hexadecanoyl-D-glucopyranose; et leurs mélanges. On peut notamment préparer les esters de glucose de la vitamine F grâce à ce procédé.
Pour mémoire, on considère que la vitamine F est généralement constituée (% en poids) :
- de 75 à 80% en poids d'acide linoléïque, et
- de 10 à 15% en poids d'acide oléïque,
- de 4 à 8% en poids d'acide palmitique,
- de 0,5 à 3% en poids d'acide stéarique, et
- de 0 à 10% en poids d'un ou plusieurs autres acides choisis parmi les acides laurique, myristique, arachidique, béhénique, lauroleïque, myristoleïque, palmitoleïque et linolénique.
Il en résulte que l'invention peut permettre de préparer par estérification de la vitamine F, un produit qui est constitué d'un mélange de différents esters, découlant de la présence des différents acides, formés lors de cette réaction.
On a constaté que, d'une manière générale, avec le procédé selon la présente invention, le glucose était estérifié principalement en position 6, et éventuellement en position 3, et/ou en d'autres positions.

### Exemple : préparation de l'ester de glucose de la vitamine F (majoritairement ester en position 6)

Dans un réacteur inerté à l'azote de 1 litre, on coule 18,7 g de chloroformiate d'isopropyle en solution 0,15 M dans le toluène (153 ml). On ajoute, sous atmosphère inerte et à 0°C, un mélange de 38,9 g de vitamine F et 15,5 g de triéthylamine préalablement mis en solution dans 45 ml de toluène; on laisse sous agitation pendant 1 heure à 20°C puis on filtre les sels formés pour obtenir une solution.
Dans un réacteur de 2,5 litres, on dissout à chaud 100 g de D-glucose dans 1 litre de pyridine, et on y ajoute la solution précédente, sous atmosphère inerte, à température ambiante (20°C). On laisse le mélange sous agitation pendant 4 heures à 20°C.
Le milieu réactionnel est évaporé à sec, sous vide pour éliminer la pyridine, puis la pâte obtenue est extraite (mélange eau/solvant organique) et la phase organique récupérée est séchée, filtrée et évaporée.
On obtient 47,5 g d'une pâte jaune d'ester de vitamine F, dont 67% de monoesters (mélange) en position 6.

Les spectres RMN ¹H et ¹³C (DMSO) 200MHz sont conformes à la structure attendue.

## Revendications

1. Procédé de préparation de dérivés O-acylés du glucose, notamment majoritairement en position 6, de formule (1) : 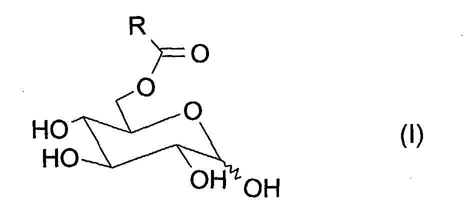 dans laquelle R est une chaîne hydrocarbonée linéaire ou ramifiée, saturée ou insaturée, comprenant 7 à 21 atomes de carbone, consistant:
- dans une première étape, à préparer un anhydride mixte de formule (II) : 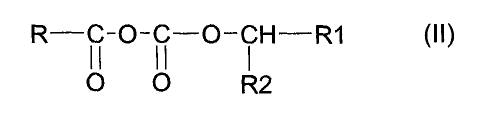
dans laquelle R1 et R2 sont, indépendamment l'un de l'autre, des radicaux hydrocarbonés, linéaires ou ramifiés, saturés ou insaturés, comprenant 1 à 20 atomes de carbone,
par réaction d'un acide carboxylique de formule R-COOH avec un halogénoformiate d'alkyle de formule X-C(O)-O-CHR1R2, avec X représentant un halogène, de préférence le chlore ou le brome;
- dans une seconde étape, à faire réagir ledit anhydride mixte formé avec le glucose.

2. Procédé selon la revendication 1, dans lequel le radical R est une chaîne hydrocarbonée linéaire ou ramifiée, saturée ou insaturée, comprenant 11 à 17 atomes de carbone.

3. Procédé selon l'une des revendications précédentes, dans lequel le reste acyle -COR est un reste choisi parmi les restes octanoyle, décanoyle, dodécanoyle, myristoyle, hexadécanoyle, stéaroyle, palmitoléoyle, oléoyle, linoléoyle ou linolénoyle.

4. Procédé selon l'une des revendications précédentes, dans lequel l'halogénoformiate d'alkyle est choisi parmi les composés pour lesquels R1 et/ou R2 sont, indépendamment l'un de l'autre, des radicaux hydrocarbonés, linéaires ou ramifiés, saturés ou insaturés, comprenant 1 à 6 atomes de carbone, notamment R1 et/ou R2 sont choisis parmi méthyle ou éthyle, et plus particulièrement les composés X-C(O)-O-CH(CH₃)₂, ou halogénoformiates d'isopropyle, et en particulier le chloroformiate d'isopropyle.

5. Procédé selon l'une des revendications précédentes, dans lequel la première étape réactionnelle est effectuée dans un solvant organique réactionnel, tel que le tétrahydrofuranne, le N-méthylpyrrolidone, la pyridine, le toluène et leurs mélanges; de préférence dans le toluène.

6. Procédé selon l'une des revendications précédentes, dans lequel la première étape réactionnelle est effectuée à une température de -25°C à +40°C, de préférence -10°C à +10°C, et pendant une durée de 5 minutes à 5 heures, notamment de 30 minutes à 3 heures.

7. Procédé selon l'une des revendications précédentes, dans lequel la seconde étape réactionnelle est effectuée dans un solvant organique, qui peut être le tétrahydrofuranne, le N-méthylpyrrolidone, la pyridine, le toluène et leurs mélanges; de préférence la pyridine.

8. Procédé selon l'une des revendications précédentes, dans lequel la seconde étape réactionnelle est effectuée à une température proche de la température ambiante, par exemple à une température comprise entre 10°C et 40°C, de préférence 15°C à 30°C, et encore mieux 18°C à 25°C.

9. Procédé selon l'une des revendications précédentes, dans lequel la seconde étape réactionnelle est conduite pendant une durée de 1 à 15 heures, notamment de 2 à 8 heures.

10. Procédé selon l'une des revendications précédentes, pour la préparation industrielle des dérivés O-acylés du glucose, majoritairement en position 6, seuls ou en mélange, notamment des composés suivants : le 6-O-octadeca-9,12-dienoyl-D-glucopyranose; le 6-O-octadeca-9-enoyl-D-glucopyranose; le 6-O-octadecanoyl-D-glucopyranose; le 6-O-hexadecanoyl-D-glucopyranose; et leurs mélanges; en particulier des esters de glucose de la vitamine F.
